Europäisches Patentamt

**European Patent Office**

Office européen des brevets

(11) Publication number: **0 163 543**
**A2**

(12) # EUROPEAN PATENT APPLICATION

(21) Application number: **85303844.6**

(22) Date of filing: **31.05.85**

(51) Int. Cl.⁴: **A 61 K 35/26**

(30) Priority: **01.06.84 US 616160**
**16.05.85 US 734610**

(43) Date of publication of application:
**04.12.85 Bulletin 85/49**

(84) Designated Contracting States:
**AT BE CH DE FR GB IT LI LU NL SE**

(71) Applicant: **VXR, Inc.**
**217 Read Street**
**Portland Maine 04103(US)**

(72) Inventor: **Goldfarb, Marcia F.**
**9 Bowdoin Street**
**Portland Maine 04102(US)**

(72) Inventor: **DeCarolis, Richard J.**
**11 Orono Road**
**Portland Maine 04102(US)**

(72) Inventor: **Miller, Harold C.**
**647 Sunset Lane**
**East Lansing Michigan 48823(US)**

(74) Representative: **De Minvielle-Devaux, Ian Benedict**
**Peter et al,**
**CARPMAELS & RANSFORD 43, Bloomsbury Square**
**London WC1A 2RA(GB)**

(54) **Marrow cell stimulator.**

(57) A biologically active composition isolated from thymus tissue, capable of enhancing the growth of pluripotent and lymphopoietic cells in bone marrow culture systems, comprising a factor which is heat stable to approximately 80°C, which has a high charge-to-mass ratio, and which is essentially free of serum albumin. A process for isolating the composition from thymus tissue is described together with compositions comprising the biologically active composition for use as a supplement for *in vitro* cultures, such as bone marrow cultures and as a therapeutic composition from bone marrow dysfunction and for use in bone marrow transplant procedures. An antibody to the biologically active composition is described together with therapeutic and diagnostic reagents comprising same.

EP 0 163 543 A2

MARROW CELL STIMULATOR

FIELD OF THE INVENTION

This invention relates to a new composition of matter, and more particularly to a novel factor isolated from mammalian thymus tissue. This thymic factor, termed Marrow Cell Stimulator or MCS, supports the long term growth of pluripotent bone marrow cells in in vitro culture systems.

BACKGROUND OF THE INVENTION

Understanding the growth and regulation of bone marrow cells is of great importance because bone marrow contains the self-renewing, pluripotent stem cells which give rise to the divergent lines of blood cells: erythrocytes, platelets, granulocytes (neutrophils, eosinophils, and basophils), macrophages, and lymphocytes (diverse B-cells and T-cells). Certain diseases--such as the leukemias, AIDS, allergies, and others--result from an absence, surplus or imbalance of lymphoid cell populations which originate in the bone marrow. Immune system imbalances may result from defects in the growth and differentiation of various lymphocyte populations that derive ultimately from the bone marrow. The ability to grow bone marrow cells in vitro makes it possible to study the regulation of bone marrow cell differentiation. Bone marrow cell cultures also provide a source of potential transplants for treating diseases such as leukemia and congenital immune deficiency.

Hemopoiesis is the process by which undifferentiated cells of the bone marrow develop into the various blood cell types. The bone marrow of adult mammals contains self-renewing pluripotent stem cells (termed CFU-S) which can proliferate or differentiate into the progenitors of the diverse blood cell lines. Such progenitor cells proliferate or differentiate into cells with still more restricted potencies, and so on until the terminally differentiated effector cells of the various lines are produced.

The goals of bone marrow cell culture systems are to mimic in vitro the events of in vivo bone marrow cell proliferation and differentiation. For over two decades attempts have been made to determine the essential regulatory factors whose addition to the culture medium would create the appropriate microenvironment for CFU-S proliferation and/or differentiation. If such regulatory factors could be identified and purified it should be possible to both maintain replicating pluripotent CFU-S cells indefinitely and to control their differentiation into preselected developmental pathways.

Bone marrow culture systems in the prior art fall into two general categories, solid and liquid. The solid, agar systems have proven unsatisfactory because sustained hemopoiesis cannot be maintained. In 1966 two groups independently reported that mouse bone marrow cells would form colonies of granulocytes and macrophages when plated in soft agar containing horse serum, Fischer's salts, and an appropriate source of colony stimulating factor (CSF). See: Bradley et al., Aust. J. Exp. Biol. Med. Sci., 44: 287 (1966); Pluznick et al., Exp. Cell Res. 43: 553 (1966). In such systems differentiation of committed progenitor cells continues for 2 to 3 weeks but hemopoietic stem cells proliferate for only a week to ten days. Dexter, T.M. et al., Meth. Cell Biol. 14: 387 (1976).

Attempts to sustain continuous and normal hematopoiesis in vitro have been far more successful using liquid culture systems. The first somewhat successful method of maintaining CFU-S proliferation in culture involved the co-culturing of thymus cells with bone marrow cells. For example, suspensions of murine thymus cells were incubated at 37°C in Fischer's medium + 20% horse serum. After several days the cultures reportedly segregated into a population of cells adhering to the culture vessel surfaces and a population of cells suspended in the overlying medium. After the overlying medium was poured off, the cultures of adherent thymus cells were inoculated with bone marrow cells. After two weeks such thymus/bone marrow co-cultures became one of two types: one producing primarily granulocytes (G-type) or one producing primarily macrophages (M-type). In the M-type cultures the number of CFU-S, in the overlying medium, reportedly decline and are gone by the fifth week. In the G-type cultures, CFU-S reportedly persist for over 12 weeks. See: Dexter et al., J. Cell Physiol. 82: 461 (1973); Dexter et al., Meth. Cell Biol. 14: 387 (1976).

The thymus/bone marrow co-culture experiments were apparently not pursued, as that technique was soon supplanted by culture systems in which bone marrow cells were added to an established bone marrow culture. See: Dexter et al., Br. J. Haematol. 28 525 (1974); Dexter et al., J. Cell Physiol. 91: 355 (1977); Moore et al., Transp. Proc. X: 83 (1978). Modifications of such long-term mouse bone marrow cultures can reportedly generate pluripotential CFU-S for up to one year. The CFU-S present in the second inoculum migrate to

- 4 -

0163543

the adherent layer and form membrane-functional complexes with cells in the adherent layer. In the absence of the adherent layer sustained hemopoiesis will not occur. Only adherent layers from bone marrow cells can sustain hemopoiesis. Adherent layers formed from spleen or thymus tissue do not sustain active hemopoiesis. Dexter et al., J.Supramol. Struct. 13: 501 (1980). Few, if any, factors are released into the medium from the adherent layer. If diffusion chambers are placed on preformed adherent layers, hemopoiesis is not maintained. Bently, S.A., Exp. Haematol. 8: 77 (1981).

Human bone marrow cultures have, by modification of the Dexter technique, been maintained for as long as 20 weeks. See: Moore et al., Blood 55: 682 (1980); Greenber et al., Blood 58: 724 (1981).

## BRIEF DESCRIPTION OF THE INVENTION

The novel thymic extract of the present invention--called Marrow Cell Stimulator or MCS--supports the long-term growth of pluripotent bone marrow cells in in vitro culture systems. MCS is extracted from neonatal vertebrate thymus tissue by means of a two-part procedure. A crude thymus extract is first prepared in conformance with procedures known in the art. Said crude extract is then subjected to a preparative electrophoretic enrichment protocol, preferably using a "powder block" electrophoresis apparatus. The fastest running material obtained by this electrophoretic procedure-- the material which precedes bovine or other animal serum albumin--is collected and concentrated to give MCS.

## DETAILED DESCRIPTION OF THE INVENTION

The marrow cell stimulator (MCS) of the present invention is isolated from calf or other neonatal vertebrate tissue by a novel procedure. Calf thymus tissue is preferred because of its relative availability. Considering this isolation procedure in detail, thymus glands obtained from freshly slaughtered animals are first freed of connective tissue and other extraneous tissue, then minced. Next an excess of homogenization buffer, for example from a 1-fold to a 3-fold excess (V/W), and preferably at least a 2-fold excess, at a pH of from about 7.5 to 8.2, and preferably of about 8.0, is added to the minced glands. A suitable homogenization buffer can be prepared, for example, by adding 0.05 moles sodium chloride and 0.0005 moles of magnesium chloride per 1 L of 10mM Tris-HCl (pH 8.0). The minced glands-buffer mixture is then homogenized (for example in a commercial grade Waring blender at 4°C for 30 seconds at maximum output), and the thus-obtained homogenate is centrifuged for from about 20 minutes to about 40 minutes at 4°C and 12,000 x g.

The supernatant liquid is then removed and heated until it has congealed. This will usually take from about 30 to about 60 minutes, preferably from about 40 to about 50 minutes, at a temperature of from about 75°C to about 85°C. The solids are then removed from the congealed supernatant liquid by again centrifuging for from about 20 minutes to about 40 minutes at 4°C and 12,000 x g. The resulting supernatant liquid is then frozen in 200-300 aliquots at -80°C for later use in the remainder of the procedure.

The electrophoretic step can be carried out by first thawing 300 mls of frozen thymus extract (ordinarily, the aliquot will be completely thawed by simply allowing it to stand at 4°C overnight; if further thawing is necessary, the temperature can be raised to 37°C), then centrifuging the thawed extract for from about 20 minutes to about 40 minutes at 4°C and 40 - 50,000 x g, and then concentrating the supernatant obtained 60-fold in an Amicon stirred cell fitted with a PM-10 membrane (which will pass molecules of molecular weight less than 10,000 daltons).

Next, the concentrate is dialyzed in a cellulose dialysis tube having a pore size which will pass molecules of molecular weights less than 12-14,000 daltons, by suspending the tube in 1 liter of BioRad "Laurell" barbital/calcium lactate buffer for 16 hours at 4°C, and then redialyzing in 1 liter of fresh "Laurell" buffer. This provides a 1:40,000 v/v exchange with the barbital/calcium lactate buffer.

The dialyzed concentrate is then admixed with a small amount (e.g., about 4 or 5 drops) of 3% bromphenol blue, which acts as a tracking dye, and then admixed with sufficient dry "Pevikon" plastic beads (Mercer Chemical Co.) to form a blue, spreadable paste.

An electrophoresis block (approximately 41 cm x 17.8 cm x 1.9 cm) of "Pevikon" is prepared by first suspending 1700 ml of the beads in two liters of distilled water and allowing the beads to settle (this will usually take at least 5 hours), then pouring off the water and "fines" next resuspending the beads in two liters of BioRad "Laurell" buffer and allowing the beads to settle, and finally pouring the Pevikon into the casting block and cutting a trough about 2.5 - 3.5 cm from the sides of the block, about 8 cm from the "back" edge and about 0.6 - 1 cm thick, leaving about a 1 mm cover of Pevikon on the bottom of the trough.

The blue paste is packed into the trough, the wicks are bathed in tanks containing 2000 ml of Laurell buffer, the necessary electrical connections are made, and electrophoresis is begun at 200 volts ($\cong$ 40 milli Amps) for from about 19 to about 21 hours, the temperature being maintained at 4°C throughout. At this point, the run is interrupted to replace the buffer in the electrode tanks with a fresh batch and to clean collected salt from the anode, following which electrophoresis is begun again at 200 volts ($\cong$ 45 milli Amps) for from about 7 to about 9 hours, then continued at 150 volts ($\cong$ 30 milli Amps) for from about 15 to about 17 hours, and concluded at 200 volts until albumin migration has reached approximately 18.5 cm, the temperature again being maintained at 4°C throughout.

The block is then separated from its electrical connections and, starting at the anode, the Pevikon is cut into 22 fraction strips, each 0.66 cm thick (fraction 22 will be well into the albumin line). Each fraction strip is then packed into a soft, punctured centrifuge tube, with the punctured end mesh-covered to retain the solid contents, each tube is placed in a 50 cc Nalgene concical centrifuge tube, and all the tubes are centrifuged for 20 minutes at 5000 rpm at 4°C. The liquid from each tube is poured into a separate bottle, 5 ml of deionized water is added to the Pevikon beads in the centrifuge tubes, and the tubes are centrifuged for 20 minutes at 5000 rpm at 4°C. The liquid from each tube is then added to that previously collected. This washing step is then repeated twice more, and the bottles containing all the liquid collected from each tube are stored at 4°C.

Each of these 22 liquid fractions is run in an Ouchterlony assay against anti-bovine serum albumin, and the fractions which result in non-positive wells, indicating no trace of albumin, are pooled. This will usually be fractions 1 - 16.  This fastest running material (that migrated ahead of albumin in the electric field) is what is referred to as MCS.  Thus, MCS can be characterized as having a high charge-to-mass ratio and as being essentially free of serum albumin.

The aforementioned pooled fractions are concentrated by standard techniques, first by ultrafiltration to a volume of a proximately 1.0 ml in an Amicon stirred cell fitted with a YM-5 membrane, then by dialysis against phosphate buffered saline (PBS).  The resulting concentrate contains the isolated MCS factor.

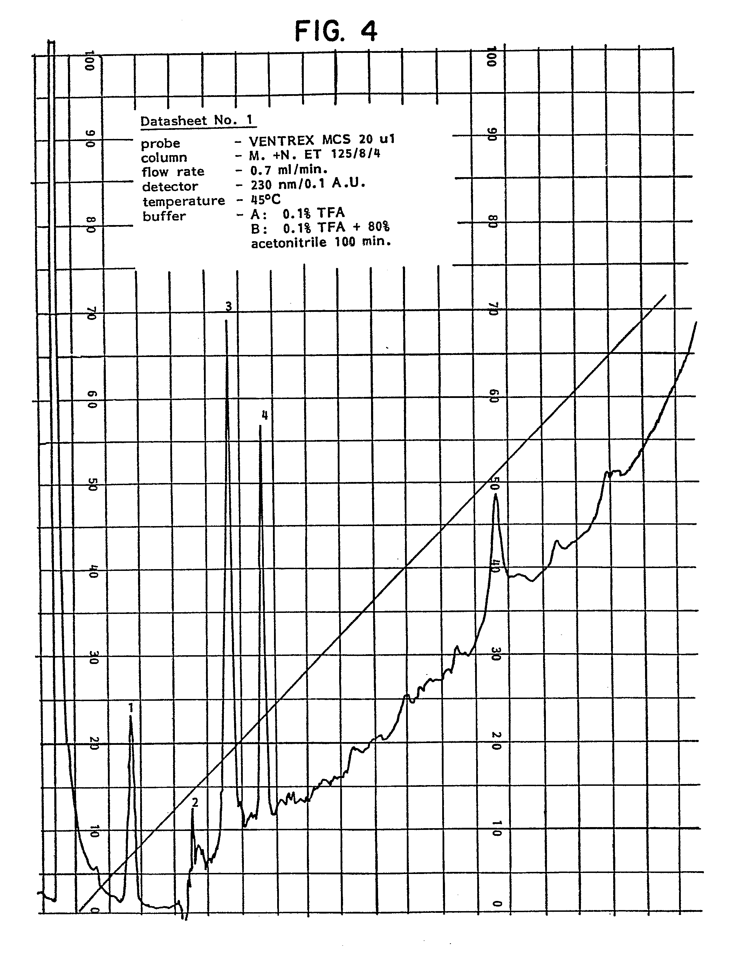

To further characterize MCS the concentrate was subjected to reverse phase high performance liquid chromatography (RP-HPLC).  An aliquot was taken up in 0.1% trifluoroacetic acid, centrifuged and the supernatant examined in a Macherey and Nagel RP-HPLC protein column.  Three probes were run under the same conditions, as shown on the legend of Figs. 4 - 6, varying only the slope of the solvent gradient and the detection wave length.

Fig. 4 shows the analytical run monitored at 230 nm. The acetonitrile gradient was generated within 100 min from 0% to 80%.  The straight line in the chromatogram represents the slope of the gradient.

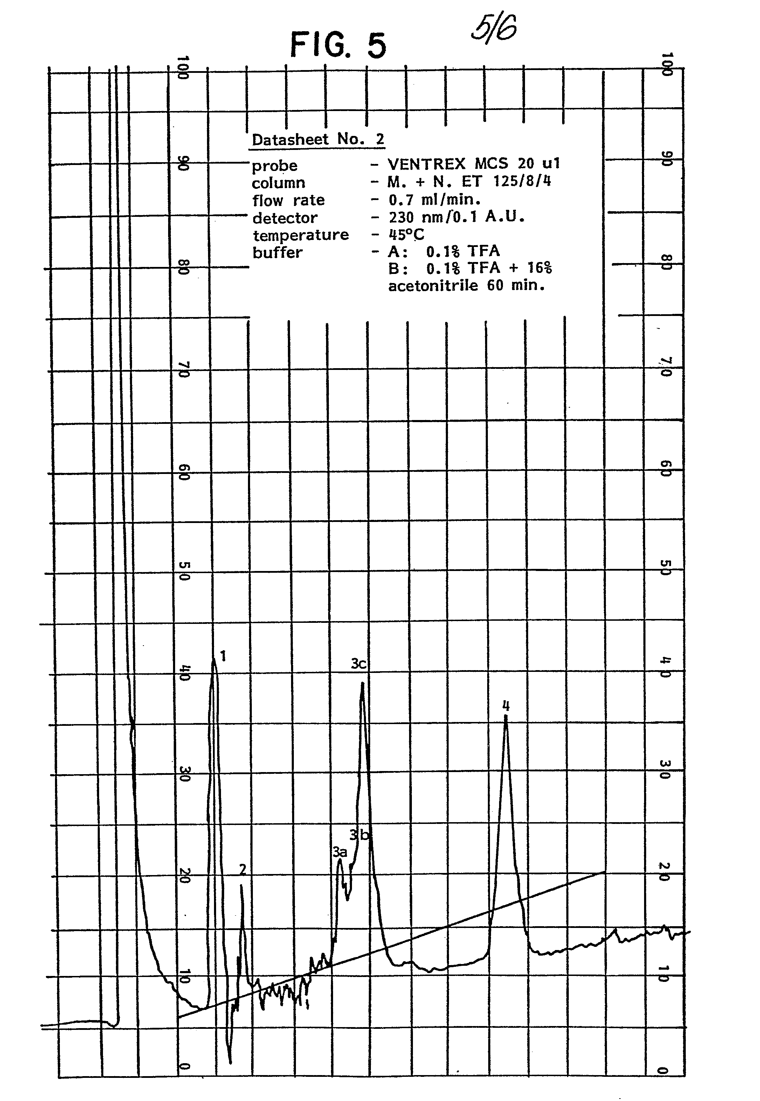

Under conditions stated above, it is possible to resolve the MCS probe into 5 main peaks.  Peak 0 represents the void volume of the column.  With a shallower gradient (Fig. 5) —0% to 16% acetonitrile within 60 min - peak 3 splits into three subpeaks, designated as 3a, 3b and 3c.       —

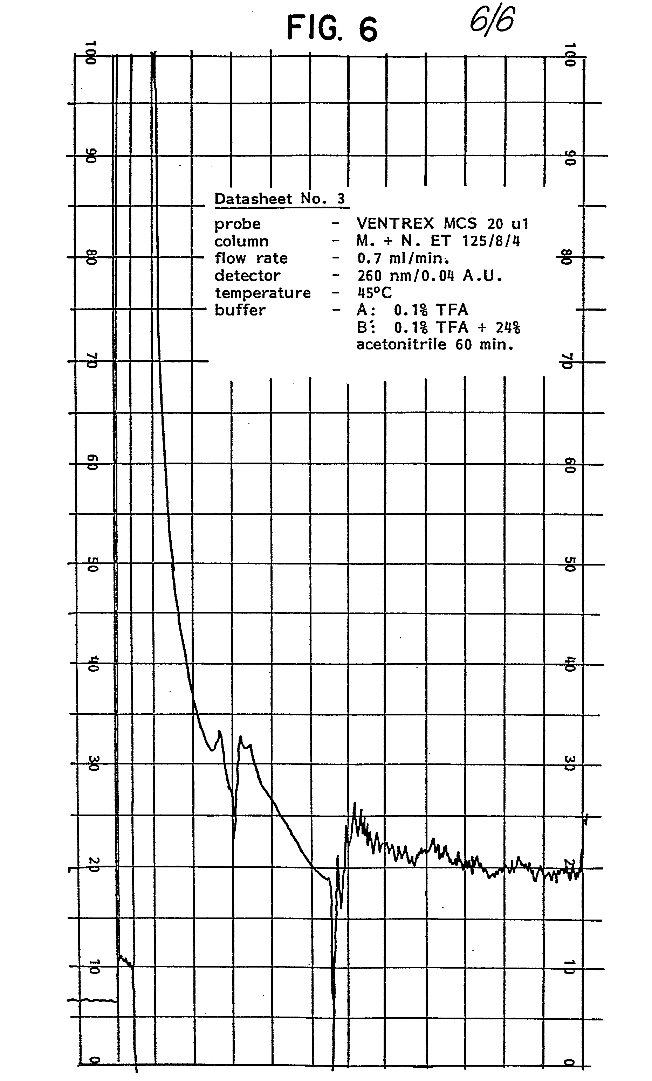

Fig. 6 shows a run monitored at 260 nm.  No definite retarded peak could be detected, indicating that peaks 1, 2, 3 and 4 have no absorptions at this wavelength.  Only the void volume peak was increased indicating that the substance which has the high absorption in the Pevikon pool elutes in the void volume.

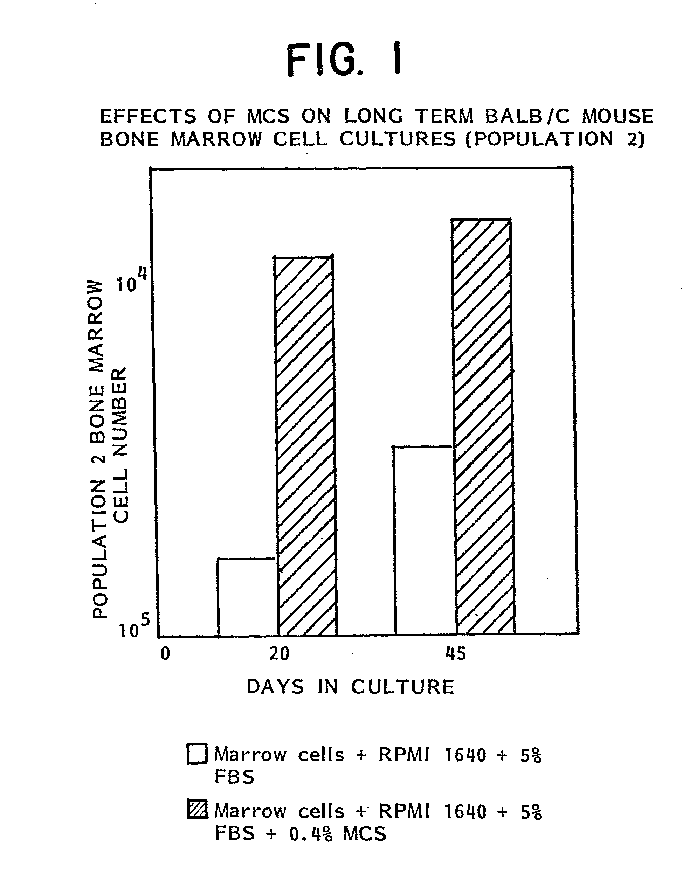

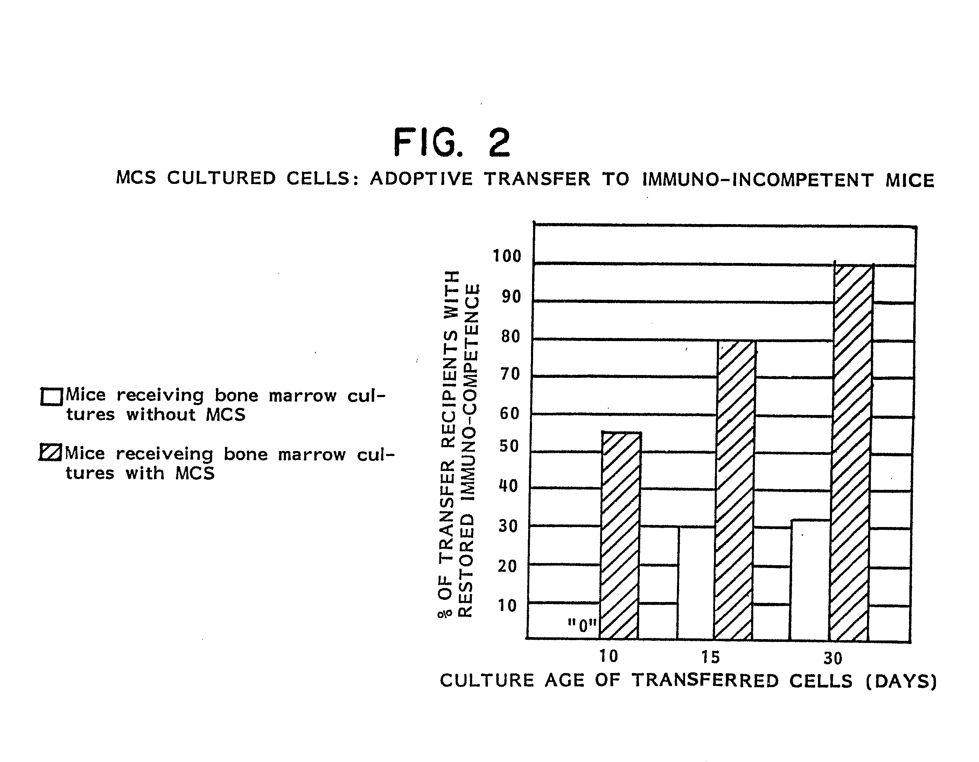

MCS, when added to <u>in vitro</u> bone marrow culture systems, supported the long-term growth of a population of pluripotent bone marrow cells for at least 45 days, as shown by Figure 1. In Fig. 1, "FBS" signifies fetal bovine serum and "RPMI 1640" signifies a common commercially available defined nutrient medium from Gibco. Furthermore, similar data were obtained from bone marrow cell cultures incubated with MCS for 90 days. Cells from said 45 and 90 day cultures are capable of reconstituting the lymphoid, myeloid and erythroid systems of lethally irradiated mice, as illustrated in Fig. 2. This indicates that a pluripotent cell population must be present in the MCS-cultured cells. Bone marrow cell cultures grown without MCS were observed to lose this reconstituting ability within the first 25 - 30 days of culture.

The ability of cells from MCS-supplemented bone marrow cultures to fully reconstitute lethally irradiated mice clearly demonstrates the presence of a pluripotential population within these cultures. Further evidence of the increased presence of a lymphopoietic population can be seen in an elevated level of terminal deoxynucleotidyl transferase (TdT) marker in cultures grown with MCS. This marker is known to be present on most immature lymphoid cells, and the elevated presence of TdT in marrow cell cultures grown with MCS may be viewed as an indicator of increased lymphopoietic ability.

In longer term culture of normal marrow cells (without MCS) and MCS-treated marrow, conducted side by side, normal marrow cells alone were found to survive only 60 - 90 days, whereas the MCS-treated marrow cells·continued to produce pluripotent stem cells up through 320 days of observation.

It was found advantageous in enhancing the reproducibility of the culture conditions, to grow the MCS-treated marrow cells in the same medium used for the experiments depicted in Fig. 2, but on gelatin beads. Especially preferred gelatin beads were those so.. under the trade name "Ventregel".

MCS can be used as a culture medium supplement to support growth of marrow cells for in vitro study of immunoregulation and bone marrow function. MCS can also support the growth in vitro of bone marrow cells used in clinical marrow cell transplant procedures and other therapeutic or diagnostic procedures.

A wide range of MCS concentrations can be used for in vitro culture to achieve general multiplication of select differentiation for myeloid, erythroid or lymphoid cells. Readministrations of MCS to cultures at time of feeding and perhaps every 2 - 6 days will enhance cellular activity in subsequent yields of pluripotential or unipotential marrow cell populations.

It has also been found that MCS-treated marrow cells produce a lymphopoietic factor, and that cultivation of fresh marrow cells with spent medium containing this factor induces the expression of pre B lymphocytes and B cells. This is illustrated in the following table, wherein in each instance fresh marrow cells were grown for seven days on the identified spent medium:

| Spent Medium Source--(15-day cultures) | Pre B lymphocytes (cytoplasmic IgM) $x\ 10^{-6}$ | B lymphocytes (surface IgM) $x\ 10^{-6}$ |
|---|---|---|
| Normal marrow cells on Ventregel (no MCS) | $54 \pm 12$ | $21 \pm 10$ |
| MCS-treated marrow cells on Ventregel | $429 \pm 173$ | $51 \pm 21$ |
| Normal marrow cells (no MCS; no Ventregel) | $3 \pm 2$ | 0 |

The data in the table were obtained by (i) incubating an aliquot with fluorescent anti-IgM at 4°C and then counting cells with surface IgM in a cell sorter and (2) incubating another aliquot with fluorescent anti-IgM at 37°C for 1 hour followed by fixation in para-formalin and counting the cells with cytoplasmic IgM in a cell sorter.

Following animal studies, it is anticipated that MCS will be applied in human clinical treatment. Deficiencies in marrow-derived progeny may be repaired by removing marrow cells, culturing with MCS, alone or in combination with other modulatory factors, to achieve the numbers of select cell types required to permit therapeutic readministration to the patient.

In addition, selected cloned populations may be retained and propagated in vitro for subsequent administration. Severe immunodeficiencies or bone marrow diseases may be repaired e.g. by the above procedure but with pluripotential cells from a relative or even unrelated person. Following MCS supported growth, peer populations of primordial cells which usually lack or have sparse histocompatability proteins may be transfused and thus not rejected. Once they generate new lymphoid populations in their chimeric recipients, they will tolerate the person as did our own lymphocytes when they first brushed against other cell types during early fetal life.

As another more complex step, cancer, organ transplants and autoimmune diseases which have been repaired as much as possible by medical and/or surgical treatments may next receive new pluripotential cells from MCS treated cultures of self or non-self origin.  The latter may be transplanted to the recipients after their residual lymphocytes are depleted via c,clorporin, irradiation, specific monoclonal antibody immunotargeting or combinations of these various procedures.

Monoclonal or polyclonal antibodies to MCS can provide unique and exclusive probes for determining serum levels of the MCS cytokine in young, old, normal or diseased individuals. The antibody may detect MCS in blood samples with a variety of enzyme-linked, $I^{125}$ or immunodiffusion assays.  The same approach permits monitoring of transplantation of MCS-producing tissues or determination of the indirect or direct effects of pharmacologic agents on these sites.  Likewise, during repair of other disease states, MCS levels can provide important clues as to the prognostic or predicted status of the patients by daily monitoring of blood samples.

## EXAMPLE 1

Marrow Cell Stimulator (MCS) was prepared as described above and was used as a direct media supplement (0.4% v/v) in standard culture medium during initial plating and early growth stages of fresh bone marrow cultures.  Bone marrow cells were obtained from femurs of 4 week old BALB/c mice and were suspended in RPMI 1640 medium from Gibco with 5% FBS (fetal bovine serum), 0.05 mM 2-mercaptoethanol and antibiotics at a cell density of $1 \times 10^6$ nucleated cells/ml.  MCS (0.4% v/v) was present at the initial plating and for the first 3 days of culture.

MCS-supported and control cultures were compared by cell sorter analysis. Cell sorting by dual laser flow cytometry has allowed the imaging of distinct cell populations within murine bone marrow and has demonstrated that pluripotent and lymphopoietic cells are almost entirely contained within one discrete sub-population, here called Population 2. Miller, H. et al, 1984, "Cell Sorter Analysis and Separation of B-Lymphoid Population from Murine Bone Marrow" (in press). Further analysis has shown that the growth of this sub-population is significantly enhanced by the presence of MCS. See Figure 1.

### EXAMPLE 2

The ability of MCS-supported bone marrow cultures to reconstitute the immune system of lethally irradiated mice was investigated by adoptive transfer studies in which cells were grown as previously described and then adoptively transferred such that the control group received $5 \times 10^6$ cultured bone marrow cells of varying culture age. A second group of mice received the same number of MCS-supported cultured cells. Both were adoptively transferred by i.v. injection (Dorshkind, K., and R.A. Phillips, 1982, J. Immunol. 129: 2444). Prior to injection, both groups were lethally irradiated with 900 rads of total body irradiation from a Cobalt 60 source. Restored immunocompetence was measured by the ability to mount an immune response of greater than 5000 anti-SRBC, PFC per spleen at day 30 (Mishell, B. and S. Shiigi, 1980, Selected Methods in Cellular Immunology, W.H. Freeman and Co., San Francisco). See Figure 2.

- 14 -

## EXAMPLE 3

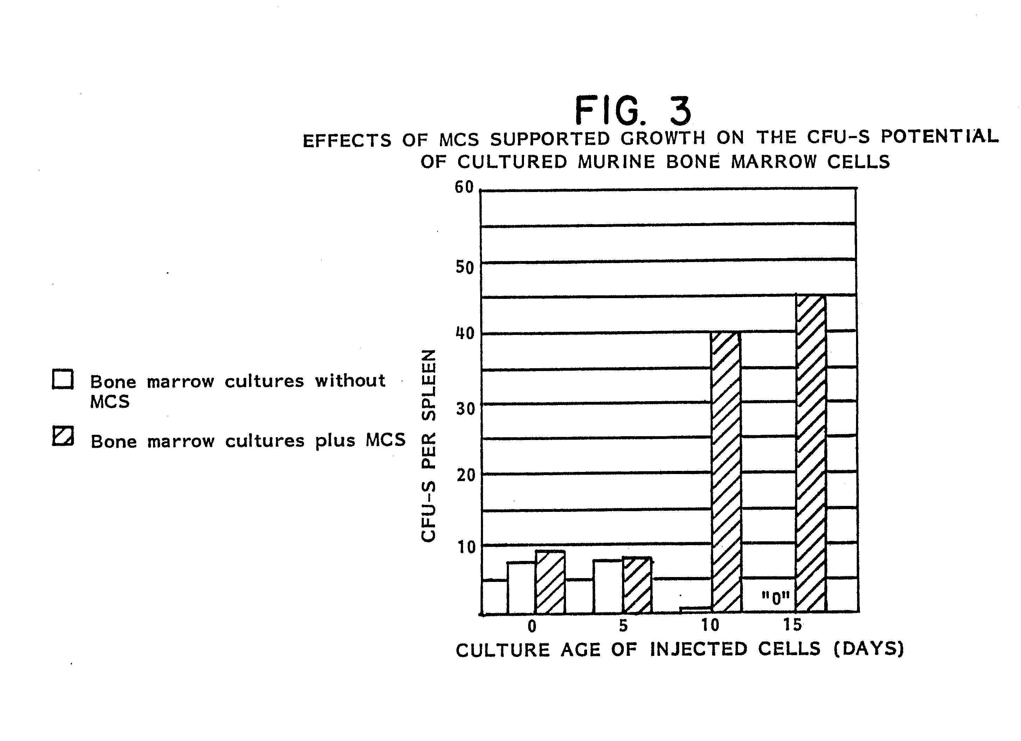

The CFU-S assay was used as a measure of the clonogenic potential of adoptively transferred bone marrow cells. $10^5$ cultured cells (same conditions as previous examples) were injected i.v. into lethally irradiated mice. Spleens were excised at day 7 and placed in fresh Boulin's fixative. The spleens were then macroscopically examined for the presence of surface colonies (Till, J.E. and E.A. McCulloch, 1961, Rad. Res. 14: 213). As can be seen in Figure 3, the use of MCS as a culture supplement significantly increased the CFU-S potential of marrow cells, indicating a possible elevation in the number of pluripotential bone marrow cells present in the MCS culture.

FIGURE LEGENDS

Figure 1 - Effects of MCS on long term BALB/C mouse
bone marrow cell cultures (Population 2)

☐ Marrow cells + RPM1 1640 + 5% FBS

▨ Marrow cells + RPM1 1640 + 5% FBS
+ 0.4% MCS

Figure 2 - MCS cultured cells: Adoptive transfer to
immuno-incompetent mice

☐ Mice receiving bone marrow cultures
without MCS

▨ Mice receiving bone marrow cultures
with MCS

Figure 3 - Effects of MCS supported growth on the CFU-S
potential of cultured murine bone marrow cells

☐ Bone marrow cultures without MCS
▨ Bone marrow cultures plus MCS

Figure 4 - Reverse Phase - HPLC

| | |
|---|---|
| Probe | - VENTREX MCS 20 µl |
| Column | - M. + N. ET 125/8/4 |
| Flow Rate | - 0.7 ml/min. |
| Detector | - 230 nm/0.1 A.U. |
| Temperature | - 45°C |
| Buffer | - A: 0.1% TFA |
| | B: 0.1% TFA + 80% |
| | acetonitrile 100 min. |

**Figure 5** - Reverse Phase - HPLC

| | |
|---|---|
| Probe | - VENTREX MCS 20 ul |
| Column | - M. + N. ET 125/8/4 |
| Flow Rate | - 0.7 ml/min. |
| Detector | - 230 nm/0.1 A.U. |
| Temperature | - 45°C |
| Buffer | - A: 0.1% TFA |
| | B: 0.1% TFA + 16% |
| | acetonitrile 60 min. |

**Figure 6** - Reverse Phase - HPLC

| | |
|---|---|
| Probe | - VENTREX MCS 20 ul |
| Column | - M. + N. ET 125/8/4 |
| Flow Rate | - 0.7 ml/min. |
| Detector | - 260 nm/0.04 A.U. |
| Temperature | - 45°C |
| Buffer | - A: 0.1% TFA |
| | B: 0.1% TFA + 24% |
| | acetonitrile 60 min. |

CLAIMS:

1. A biologically active composition isolated from thymus tissue, capable of enhancing the growth of pluripotent and lymphopoietic cells in bone marrow culture systems, comprising a factor which is heat stable to approximately 80°C, which has a high charge-to-mass ratio, and which is essentially free of serum albumin.

2. A composition as in claim 1, isolated from calf thymus tissue.

3. A process for isolating a marrow cell stimulator composition from thymus tissue, the steps of which comprise:

A. preparing a crude thymic extract;

B. subjecting said crude thymic extract to preparative electrophoresis;

C. collecting the material which migrates faster in the electric field than does bovine or animal serum albumin, and

D. concentrating said collected material.

4. A process as in claim 3, wherein the marrow cell stimulator factor is isolated from calf thymus tissue.

5. A process as in claim 3, wherein the crude thymic extract of step A is prepared as follows:

A. removing connective and other extraneous tissues from thymus glands of freshly slaughtered neonatal vertebrates;

B. homogenizing said thymus glands in a buffered solution;

C. centrifuging the homogenate to remove cellular debris;

D. heating the supernatant from step C to approximately 80°C until soluble proteins, including proteases, have congealed;

E. removing said congealed material by centrifugation;

F.  freezing the supernatant from step E;   0163543

G.  thawing the supernatant and removing any solids by high speed centrifugation;

H.  concentrating the supernatant from step G by ultrafiltration using a membrane that retains molecules with molecular weights larger than 10,000 daltons, and

I.  dialyzing the concentrate from step H to eliminate molecules of molecular weights less than 12,000 to 14,000 daltons.

6.  A process as in claim 3, wherein a powder-block electrophoresis apparatus is employed in step B.

7.  A marrow cell stimulator composition isolated by the process of claim 3.

8.  A supplement for _in vitro_ cell cultures, comprising a composition as in either of claims 1 or 7.

9.  A supplement as in claim 8, for bone marrow cell cultures.

10.  A supplement as in claim 9, to maintain and support proliferation of a pluripotent stem cell population.

11.  A supplement as in claim 9, to maintain and support lymphopoiesis.

12.  A therapeutic composition comprising a composition as in either of claims 1 or 7.

13.  A therapeutic composition as in claim 12, for bone marrow transplant procedures.

14.  A therapeutic composition as in claim 12, for _in vivo_ treatment of immunodeficiencies resulting from bone marrow dysfunction.

15.  A therapeutic composition comprising an antibody to the composition of either of claims 1 or 7.

16.  A clinical diagnostic composition comprising an antibody to the composition of either of claims 1 or 7.

**0163543**

17. A composition as in either of claims 1 or 7 for use as a pharmaceutical.

18. A process for preparing a therapeutic composition according to any of claims 12 to 14 comprising the step of bringing a composition as in either of claims 1 or 7 into association with a pharmaceutically acceptable excipient.

19. A process for preparing a therapeutic composition according to claim 15 comprising the step of bringing an antibody to a composition as in either of claims 1 or 7 into association with a pharmaceutically acceptable excipient.

# FIG. 1

EFFECTS OF MCS ON LONG TERM BALB/C MOUSE
BONE MARROW CELL CULTURES (POPULATION 2)

☐ Marrow cells + RPMI 1640 + 5% FBS

▨ Marrow cells + RPMI 1640 + 5% FBS + 0.4% MCS

# FIG. 2

## MCS CULTURED CELLS: ADOPTIVE TRANSFER TO IMMUNO-INCOMPETENT MICE

☐ Mice receiving bone marrow cultures without MCS

▨ Mice receiveing bone marrow cultures with MCS

Y-axis: % OF TRANSFER RECIPIENTS WITH RESTORED IMMUNO-COMPETENCE (10, 20, 30, 40, 50, 60, 70, 80, 90, 100)

X-axis: CULTURE AGE OF TRANSFERRED CELLS (DAYS) — "0", 10, 15, 30

# FIG. 3

EFFECTS OF MCS SUPPORTED GROWTH ON THE CFU-S POTENTIAL
OF CULTURED MURINE BONE MARROW CELLS

□ Bone marrow cultures without MCS

▨ Bone marrow cultures plus MCS

Y-axis: CFU-S PER SPLEEN (0 to 60)

X-axis: CULTURE AGE OF INJECTED CELLS (DAYS) — 0, 5, 10, 15

"0"

3/6

0163543

## FIG. 4

Datasheet No. 1

probe — VENTREX MCS 20 u1
column — M. +N. ET 125/8/4
flow rate — 0.7 ml/min.
detector — 230 nm/0.1 A.U.
temperature — 45°C
buffer — A: 0.1% TFA
B: 0.1% TFA + 80%
acetonitrile 100 min.

FIG. 5

0163543

5/6

Datasheet No. 2

| | |
|---|---|
| probe | – VENTREX MCS 20 u1 |
| column | – M. + N. ET 125/8/4 |
| flow rate | – 0.7 ml/min. |
| detector | – 230 nm/0.1 A.U. |
| temperature | – 45°C |
| buffer | – A:  0.1% TFA |
| | B:  0.1% TFA + 16% |
| | acetonitrile 60 min. |

0163543

## FIG. 6

6/6

Datasheet No. 3

| | | |
|---|---|---|
| probe | – | VENTREX MCS 20 u1 |
| column | – | M. + N. ET 125/8/4 |
| flow rate | – | 0.7 ml/min. |
| detector | – | 260 nm/0.04 A.U. |
| temperature | – | 45°C |
| buffer | – | A: 0.1% TFA |
| | | B: 0.1% TFA + 24% acetonitrile 60 min. |